# EUROPEAN PATENT SPECIFICATION

(11) **EP 2 324 841 B1**
(45) Date of publication and mention of the grant of the patent: **11.07.2018**
(21) Application number: 08783591.4
(22) Date of filing: 29.07.2008
(51) Int. Cl.: A61K 36/25, A61K 36/324, A61K 36/65, A61K 36/54, A61K 36/185, A61K 35/64, A61K 35/62, A61K 31/045, A61P 9/10

(54) **TRADITIONAL CHINESE MEDICINAL COMPOSITION FOR THE TREATMENT OF CARDIOVASCULAR DISEASE**
TRADITIONELLE CHINESISCHE MEDIZINISCHE ZUSAMMENSETZUNG ZUR BEHANDLUNG VON KARDIOVASKULÄREN ERKRANKUNGEN
COMPOSITION DE MÉDECINE CHINOISE TRADITIONNELLE POUR LE TRAITEMENT DES MALADIES CARDIOVASCULAIRES

(43) Date of publication of application: 25.05.2011
(73) Proprietor: Hebei Yiling Medicine Research Institute Co., Ltd., Hebei 050035 (CN)
(72) Inventor: YANG, Yuejin, Hebei 050035 (CN); QIAN, Haiyan, Hebei 050035 (CN)
(74) Representative: Kolster Oy Ab
(86) International application number: PCT/CN2008/001401
(87) International publication number: WO 2010/012127

(56) References cited:
- CN-A- 1 334 121
- CN-A- 1 709 291
- CN-A- 1 939 438
- CN-A- 1 954 825
- QIAN HAI-YAN ET AL: "Effects of Tongxinluo-facilitated cellular cardiomyoplasty with autologous bone marrow-mesenchymal stem cells on postinfarct swine hearts.", CHINESE MEDICAL JOURNAL 20 AUG 2007 LNKD- PUBMED:17825171, vol. 120, no. 16, 20 August 2007 (2007-08-20), pages 1416-1425, XP002662899, ISSN: 0366-6999
- WU TAIXIANG ET AL: "Tongxinluo (Tong xin luo or Tong-xin-luo) capsule for unstable angina pectoris.", COCHRANE DATABASE OF SYSTEMATIC REVIEWS (ONLINE) 2006 LNKD- PUBMED:17054205, no. 4, 2006, page CD004474, XP002662362, ISSN: 1469-493X

## Description

### Field of the invention

The present invention relates to a traditional Chinese medicinal composition for use in the treatment or prevention cardiovascular disease. The traditional Chinese medicinal composition has promotive effects on bone marrow-derived mesenchymal stem cell survival *in vivo* and differentiation into cardiomyocytes.

### Background of the invention

Cardiovascular disease kills 12 million people which approximately accounts for 1/4 of all deaths of the whole world, every year, it becomes one of the most dangerous diseases for human health. To myocardial infarction or heart failure, traditional therapy, including medicine, intervention and surgery, could not make the deficient myocardial cell regenerate, and because cardiac muscle deficiency causes irreversible cardiac muscle remodelling, it finally leads to heart failure and death. In recent years, regenerative stem cell medicine has made a great progress on the treatment of cardiovascular disease, the technique is also called cellular cardiomyoplasty, that is, it achieves the repair of the damaged cardiac muscle by transplanting stem cells or myocardial cells, or by mobilizing peripheral blood stem cells or myeloid stem cells migrating to damaged parts of the cardiac muscle. Carrying out cellular cardiomyoplasty by transplanting stem cells, which is a feasible method to improve the hemodynamic profiles and neurohumoural disorder caused by myocardial infarction. Various previous animal experiments show that stem cell has the ability to repair myocardial cells, and can improve the perfusion of infarction and cardiac function [Schuster MD, Kocher AA, Seki T, Martens TP, Xiang G, Homma S, et al., Myocardial neovascularization by bone marrow angioblasts results in cardiomyocyte regeneration. Am J Physiol Heart Circ Physiol 2004; 287: H525-532.

Although stem cell was used for the clinical research of myocardial repair, due to the influence of ischemia/reperfusion and inflammatory factor, it resulted to the death of donor cells of regional ischemic heart, so the development of cellular cardiomyoplasty had been retarded by low survival rate of transplanted cells. The research shows that a large number of cells died after transplantation into a damaged heart, and there was significant loss of cells within 24 h, but 15% of transplanted cells survived after 12 weeks (Muller-Ehmsen J, Whittaker P, Kloner RA, Dow JS, Sakoda T, Long TI, et al., Survival and development of neonatal rat cardiomyocytes transplanted into adult myocardium. J Mol Cell Cardiol 2002; 34: 107-116) [PMID: 11851351].

Acute myocardial infarction could cause serious regional myocardial ischemia, inflammatory reaction, oxidative stress and apoptosis, which would greatly lower the survival rate of the transplanted cells. Thus, protection of regional transplanted stem cell, reduction or avoiding its death is important to clinical application. Several methods are now available for improving the survival rate of transplanted cell: (1) heat shock treatment could improve tolerance to ischemia/reperfusion damage *in vivo* for transplanted cell, improve the survival rate after transplantation to the heart (Suzuki K, Smolenski RT, Jaya-kumar J, Murtuza B, Brand NJ, Yacoub MH. Heat shock treatment enhances graft cell survival in skeletal myoblast transplantation to the heart. Circulation 2000; 102: III216-221)[PMID: 11082390]); (2) bone marrow-derived mesenchymal stem cell modified with Akt could further improve performance of infarcted heart (Mangi AA, Noiseux N, Kong D, He H, Rezvani M, Ingwall JS, et al. Mesenchymal stem cells modified with Akt prevent remodeling and restore performance of infarcted hearts. Nat Med 2003; 9: 1195-1201)[PMID: 12910262]; (3) Injected plasmid vector into regional ischemic myocardium resulted in ferroheme oxygenase-1 overexpression, reduction of the number of infiltration with mononuclear cells, and down regulation of the expression of inflammatory factor (Tang YL, Tang Y, Zhang C, Qian KP, Shen LP, Phillips I. Improved graft mesenchymal stem cell survival in ischemic heart with a hypoxia-regulated Ferroheme Oxygenase-1 vector. J Am Coll Cardiol 2005; 46: 1339-1350) [PMID: 1619885]. All of the above means are based on the level of donor cells, however the key to the fate of transplanted cells in the heart is the microenvironment of regional infarcted myocardium, so the intervention carried out for the microenvironment of infarcted myocardium may be more effective in promoting the survival of transplanted cells and producing a biological effect.

Qian et al. Chin Med J 2007;120(16):1416-1425, discloses the effects of Tongxinluo-facilitated cellular cardiomyoplasty with autologous bone marrow-mesenchymal stem cells on postinfarct swine hearts. Tongxinluo is a Chinese compound preparation which is used to treat coronary heart disease and ischemic cerebral vascular diseases.

The present invention is a further improvement on CN. Patent No. 01131203.3 and patent application No. 200410048292.2. The invention provides a novel use of a traditional Chinese medicinal composition for preparing medicine for promoting bone marrow-derived mesenchymal stem cell survival *in vivo* and differentiation into cardiomyocytes, which improve the quality of regional micro-environment by intervention therapy, and effectively promote the survival and biological effects of transplanted cells.

### Summary of the invention

The present disclosureprovides a use of a traditional Chinese medicinal composition for preparing a medicine for promoting bone marrow-derived mesenchymal stem cell survival *in vivo* and differentiation into cardiomyocytes, wherein the traditional Chinese medicinal composition is composed of the following crude drugs (by wt. portions):
ginseng 3-10
leech 3-11
ground beetle 5-10
olibanum (processed)1-5
red peony root 3-9
rosewood heart wood 1-5
sandalwood 1-5
scorpion 3-9
cicada slough 3-12
centipede 1-3
borneol 1-7
spine date seed(stir-baked) 3-10;
preferably, the traditional Chinese medicinal composition is composed of the following crude drugs (by wt. portions):
   ginseng 6
   leech 10
   ground beetle 7
   olibanum (processed)2
   red peony root 5
   rosewood heart wood 2
   sandalwood 2
   scorpion 7
   cicada slough 7
   centipede 1
   borneol 5
   spine date seed(stir-baked) 5;
      or:
      ginseng 10
      leech 8
      ground beetle 7
      olibanum (processed)2
      red peony root 5
      rosewood heart wood 2
      sandalwood 2
      scorpion 9
      cicada slough 7
      centipede 1
      borneol 5
      spine date seed(stir-baked) 5;

An object of this invention is to provide a traditional Chinese medicinal composition use in the treatment or prevention of cardiovascular disease, wherein the traditional Chinese medicinal composition is composed of the following crude drugs (by wt. portions):
ginseng 6
leech 11
ground beetle 7
olibanum (processed) 2
red peony root 5
rosewood heart wood 2
sandalwood 2
scorpion 3
cicada slough 7
centipede 1
borneol 5
spine date seed(stir-baked) 5;
   or:
   ginseng 5.5
   leech 10.375
   ground beetle 6.875
   olibanum (processed) 2.25
   red peony root 4.75
   rosewood heart wood 2.375
   sandalwood 2.25
   scorpion 6.875
   cicada slough 6.875
   centipede 1.375
   borneol 1.375
   spine date seed(stir-baked) 4.625;
   more preferably, the active ingredients of the above traditional Chinese medicinal composition are composed of the following:
      a. scorpion, leech, centipede, ground beetle, cicada slough and processed olibanum powder, which has a mean particle size of less than 100 µm;
      b. borneol powder;
      c. volatile oils extracted from rosewood heart wood and sandalwood;
      d. condensed alcohol extract of ginseng extracted with ethanol;
      e. condensed water extract, which is obtained as following: extracting the residue of rosewood heart wood and sandalwood with water after extracting component c from them, decocting red peony root and stir-baked spine date seed with water, extracting the residue of ginseng with water after extracting component d from it, filtering all of the above water extracts, blending them, then concentrating.

The invention further discloses that the medicinal preparation containing the above traditional Chinese medicinal composition as active components is capsule, tablet, pill, oral liquid, soft capsule, or guttate pill.

The disclosure also provides use of the above traditional Chinese medicinal composition for preparing a medicine for treatment of cardiovascular disease with autologous bone marrow-derived mesenchymal stem cells, preferably the cardiovascular disease is myocardial infarction, more preferably acute myocardial infarction.

The traditional Chinese medicinal composition promotes bone marrow-derived mesenchymal stem cells survival *in vivo* and differentiation into cardiomyocytes, and the traditional Chinese medicinal composition is in some embodiments provided for use in treating cardiovascular disease by combining with autologous bone marrow-derived mesenchymal stem cells, the cardiovascular preferably is myocardial infarction, more preferably is acute myocardial infarction.

In the traditional Chinese medicinal composition of the invention, Latin name and processing method of the raw materials as active component are derived from the Dictionary of Chinese Traditional Drugs (first edition, Shanghai Scientific and Technical Press, July, 1977), and Chinese Pharmacopeia (edition 2005, Chemical Industry Press).

The traditional Chinese medicinal composition of the invention may be formulated as any conventional pharmaceutically acceptable dosage forms, such as capsules, tablets, pills, oral liquids, capsules, guttate pills etc., according to conventional preparation process, for example, the preparation technology recorded in Chinese drugs pharmaceutics (Fan Biting, Shanghai Science Press, Dec.1997, 1st ed.).

The preparations of the invention may also comprise optionally conventional pharmaceutically acceptable excipients, such as fillers, disintegrants, binders, glidants, antioxidants, flavoring agents, sweeteners, and suspending agents etc. The excipients include, for example, starch, sucrose, lactose, dextrin, pregelatinized starch, crospolyvinylpyrrolidone etc. or other Chinese drugs pharmaceutically acceptable excipients (excipients of various dosage forms recorded in Chinese drugs pharmaceutics, FanBiting, Shanghai Science Press, Dec.1997, 1st ed.).

Preferably, the method of preparing the formulations of the invention is as following: cleaning 5 raw materials of the above proportion of leech, scorpion, cicada slough, ground beetle and centipede, drying at a low temperature, putting aside; extracting volatile oils of sandalwood and rosewood heart wood, putting the residue and the water solution aside; extracting ginseng by heating reflux with 70% ethanol twice, 3 hours for first time and 2 hours for the second time, combining the extracts and recovering ethanol completely; combining the residue of ginseng, the residue of sandalwood and rosewood heart wood and the water solution, adding red peony root and spine date seed (stir-baked) to it, adding water right amount to decoct twice, 3 hours for the first time and 2 hours for the second time, combining the decoction, filtering, concentrating the filtrate to a paste of relative density of 1.20-1.25 (60°C), next adding the alcohol extract of ginseng, mixing well, drying at a low temperature, crushing into fine powders; co-grinding of olibanum (processed) and 5 materials of leech etc. into fine powders; grinding borneol, and co-grinding gradually with the above fine powders until mixing well, spraying the volatile oils into the powders, mixing well, filling capsules to make into 1000 capsules.

Or, preferably the disclosed preparations are prepared as following:
a) the proportion by weight of the raw materials are: ginseng 3-10 portions, leech 3-11 portions, ground beetle 5-10 portions, olibanum (processed) 1-5 portions, red peony root 3-9 portions, rosewood heart wood 1-5 portions, sandalwood 1-5 portions, scorpion 3-9 portions, cicada slough 3-12 portions, centipede 1-3 portions, borneol 1-7 portions, stir-baked spine date seed 3-10 portions;
b) pulverization process for medicinal materials:
   selecting and washing the five worm medicines of scorpion, leech, centipede, ground beetle and cicada slough, then combining them with processed olibanum according to prescription, crushing with crusher to obtain a coarse powder which can reach above 80 mesh; superfine grinding the coarse powder by using superfine pulverizing technologies to achieve medicinal powder in size less than 100 µm; prescribing the medicinal materials under pulverization after cleaning, drying and sterilizing;
c) extraction, concentration and drying processes:
   adding water to rosewood heart wood and sandalwood, extracting volatile oils from them followed by extracting them with water, decocting red peony root and spine date seed, filtering water solution, putting aside; extracting ginseng with water after extracting it with ethanol, recovering the ethanol of alcohol solution and concentrating it to ethanol extract, filtering the water solution of ginseng and combining it with other water solutions, blending and concentrating to water extract;
d) preparation process:
   feeding of the superfines into fluid bed granulating drier, then spraying the extract of step c) to granulate; finishing the granules, adding the fine powder of borneol, spraying volatile oils extracted from rosewood heart wood and sandalwood, filling with capsule filling machine to make into capsules after mixing well.

Or, preferably the disclosed preparations are prepared as following:
a) the proportion by weight of the raw materials are: ginseng 3-10 portions, leech 3-11 portions, ground beetle 5-10 portions, olibanum (processed) 1-5 portions, red peony root 3-9 portions, rosewood heart wood 1-5 portions, sandalwood 1-5 portions, scorpion 3-9 portions, cicada slough 3-12 portions, centipede 1-3 portions, borneol 1-7 portions, stir-baked spine date seed 3-10 portions;
b) pulverization process for medicinal materials:
   selecting and washing the five worm medicines of scorpion, leech, centipede, ground beetle and cicada slough, then according to prescription combining them with processed olibanum, crushing with crusher to obtain a coarse powder which can reach above 80 mesh; superfine grinding the coarse powder by using superfine pulverizing technologies to achieve medicinal powder in size less than 100 µm; prescribing the medicinal materials under pulverization after cleaning, drying and sterilizing;
c) extraction, concentration and drying processes:
   adding water to rosewood heart wood and sandalwood, extracting volatile oils from them followed by extracting them with water, decocting red peony root and spine date seed, filtering water solution, putting aside; extracting ginseng with water after extracting it with ethanol, recovering the ethanol of alcohol solution and concentrating it to ethanol extract, filtering the water solution of ginseng and combining it with other water solutions, blending, concentrating the solution into water extract, then directly spray-drying to get spray dried powders.
d) preparation process:
   feeding of the superfines and the spray powders of step c) into fluid bed granulating drier, then spraying solvent to make into granules; finishing the granules, adding the fine powder of borneol, spraying volatile oils extracted from rosewood heart wood and sandalwood, filling with capsule filling machine to make into capsules after mixing well.

Dosage of the composition of the invention calculated from the total weight of the raw materials as active component is 0.8-3 g each time, 2-4 times daily, preferably is 1.11-2.22 g each time, three times daily.

A large amount of experimental data presented by the invention showed that using the drug group of the invention could promote cellular cardiomyoplasty carried out with autologous bone marrow-derived mesenchymal stem cell. Detection of the gene expression profiles after cardiac infarction by microarray found use low-dose of the drug group of the invention alone could result in positive changes to gene expression, including up-regulation of anti-inflammatory, anti-apoptosis, anti-fibrosis gene. Thus, it is believed that using of the drug group of the inventions for intervention could improve the regional micro-environment after acute infarction, so that significantly improve the implanted bone marrow-derived mesenchymal stem cells survive and differentiate. Therefore, the experimental data of the invention also show that using of the drug group of the invention for intervention could improve the regional internal environment after acute myocardial infarction efficiently, and promote cellular cardiomyoplasty carried out with autologous bone marrow-derived mesenchymal stem cell, thereby producing a positive impact on the clinical application of the transplantation of bone marrow-derived mesenchymal stem cell.

Also disclosed is a method for treatment or prevention of cardiovascular disorders with the traditional Chinese medicinal composition, comprising administering to patients in need thereof an effective amount of the traditional Chinese medicinal composition. Cardiovascular disease preferably is myocardial infarction, more preferably is acute myocardial infarction. Conventional approaches in the field may be used for administration.

### Description of the drawings

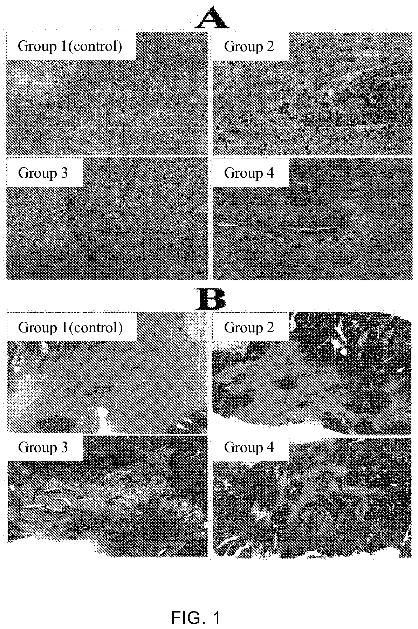

**Figure 1****. Hematoxylin-eosin(HE) staining and Masson's tri-chrome staining of infarcted areas in four group experimental animals under the microscope.**
   It shows in the pictures that the first group (the control group), the second group (treated only with low-dose of the drug of the invention for intervention), and the third group (treated only with bone marrow-derived mesenchymal stem cells transplantation for intervention) all exhibit serious fibrosis and inflammatory cellular infiltration, basically no survival cardiomyocyte is found in the infarcted areas. However, the fourth group (treated with bone marrow-derived mesenchymal stem cells transplantation combining with the drug mesenchymal stem cells transplantation combining with the drug of the invention for intervention) there were many DAPI stained transplanted cells. Figure B shows that the cellular survival potential between the third group (treated only with bone marrow-derived mesenchymal stem cells transplantation for intervention) and the fourth group (treated with bone marrow-derived mesenchymal stem cells transplantation combining with the drug of the invention for intervention) has significant statistical difference. *P<0.0001, the magnification of image A is 400×.

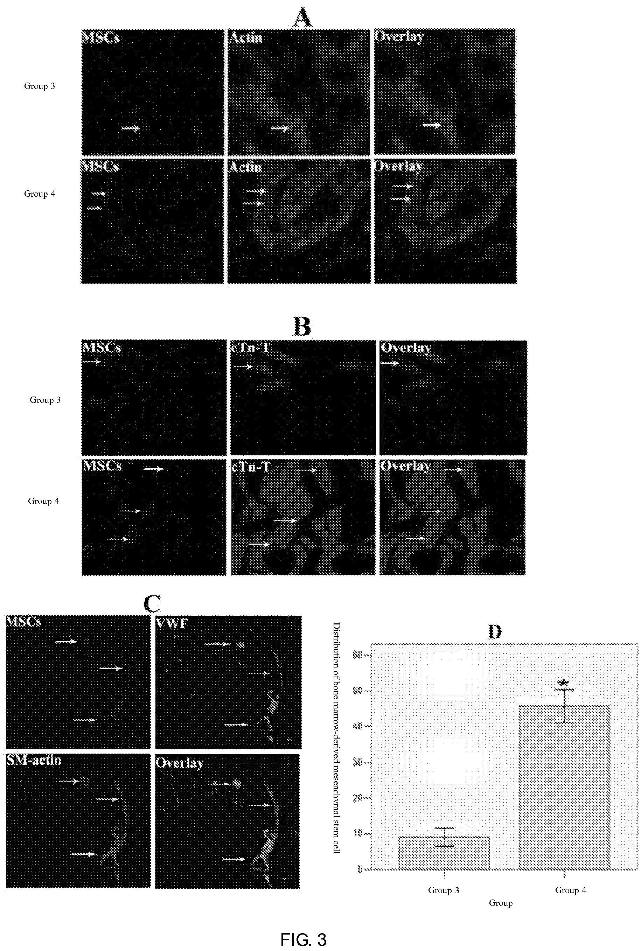

**Figure 3****. Bone marrow-derived mesenchymal stem cells trans-planted in the body differentiat into cardiomyocytes and vessel structures.**
   Figure A and Figure B show that some DAPI labelled cells express α-SCA(α-sarcomeric actin) and cTnT (Cardiac troponin T). Figure C illustrates some DAPI positive cells express VSMA(vascular smooth muscle actin) and vascular endothelial specific factor VIII, indicating the involvement of the vessel formation. It is shown in figure D that the potential of bone marrow-derived mesenchymal stem cells differentiation into cardiomyocytes of the third group (treated only with bone marrow-derived mesenchymal stem cells transplantation for intervention), when statistically compare the ratio of DAPI positive cells differentiating into cardiomyocytes with the fourth group (treated with bone marrow-derived mesenchymal stem cells transplantation combining with the drug of the invention for intervention) has significant difference. * P<0.0001, the magnification of figure A, figure B, and figure C is 400×. Note: MSCs refers to bone marrow-derived mesenchymal stem cells, VWF is von Willebrand factor, SM-actin is vascular smooth muscle actin, and Overlay refers to the staining superposition results of three visions.

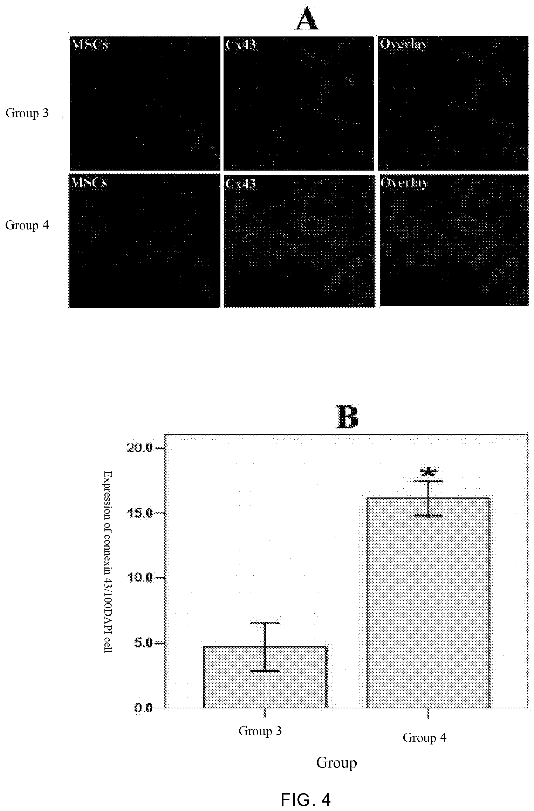

**Figure 4****. Expression of connexins in implanted cells *in vivo.***
   Figure A indicates that DAPI labelled cells express connexin 43(Cx43). Figure B shows a significant difference of the expression of connexin 43, when the third group (treated only with bone marrow-derived mesenchymal stem cells transplantation for intervention) compared with the fourth group (treated with bone marrow-derived mesenchymal stem cells transplantation combining with the drug of the invention for intervention). * P<0.0001, the magnification of figure A is 400×. Note: Overlay refers to the staining superposition results of three visions.

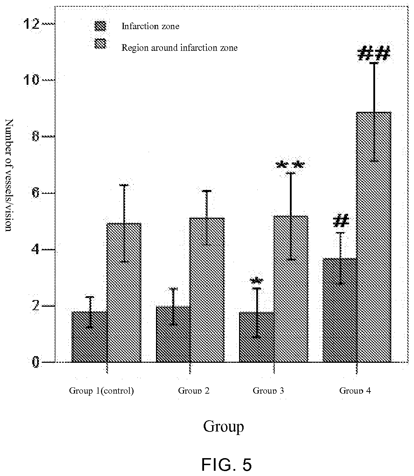

**Figure 5****. Capillary density in infarct zone and surrounding infarct zone after 6- week post-transplantation.**
   6 weeks after transplantation, the capillary density in infarct zone and surrounding infarct zone of the second group (treated only with low-dose of the drug of the present invention for intervention) and that of the third group (treated only with bone marrow-derived mesenchymal stem cells transplantation for intervention) have no significant difference from the control group (* P>0.05, ** P>0.05), but both were lower than the fourth group (treated with bone marrow-derived mesenchymal stem cells transplantation combining with the drug of the invention for intervention) (^{#} P<0.0001, ^{##} P<0.0001, respectively).

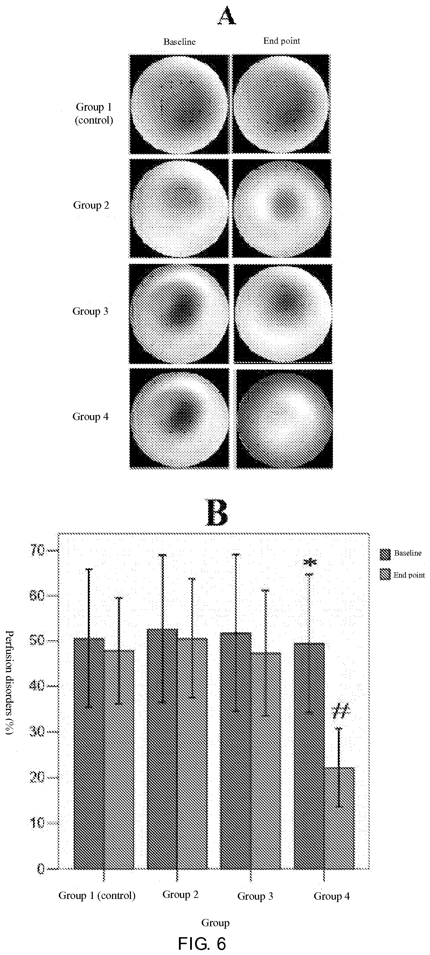

**Figure 6****. Myocardial perfusion defect area detected by single photon emission computed tomography (SPECT) 1 week and 6 weeks after transplantation.**
   Figure A shows the typical graphs of each group. Figure B: the initial SPECT results, it shows there is no significant difference among the myocardial perfusion defect area in the four groups (*P=0.984). Six weeks after transplantation, the myocardial perfusion defect area in the fourth group (treated with bone marrow-derived mesenchymal stem cells transplantation combining with the drug of the invention for intervention) decreased significantly by 22.1±9.3%, when compared with the control group, the second group (treated only with low-dose of the drug of the invention for intervention), and the third group (treated only with bone marrow-derived mesenchymal stem cells transplantation for intervention), there is a dramatic difference (n=7, ^{#}P<0.0001).

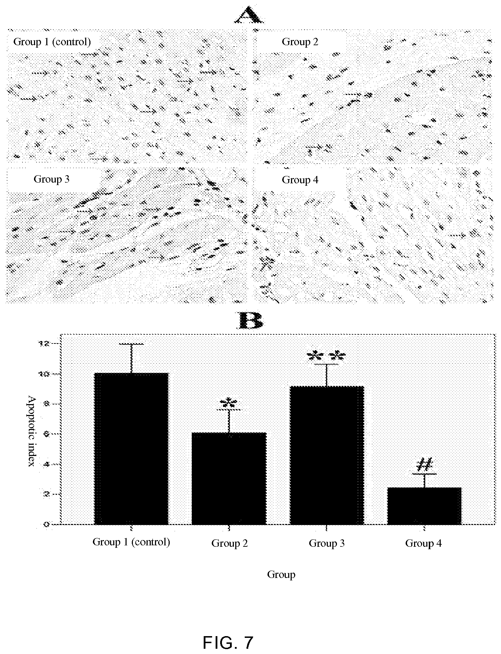

**Figure 7****. Anti-apoptotic action of the present invented drug.**
   (A) The apoptotic cells with broken DNA in the nucleus which were determined by myocardium anti-binding protein antibodies surrounding the infarct zone of pigs and terminal-deoxynucleoitidyl transferase deoxyuridine triphosphate (dUTP) mediated nick end labeling (TUNEL). At the end of the observation, there were few apoptotic nucleos in the second group (treated only with low-dose of the drug of the invention) and the fourth group (treated with bone marrow-derived mesenchymal stem cells transplantation combining with the drug of the invention for intervention) (red arrow). Magnifying is 20 times. (B) The statistical apoptosis indexes (Al) of the four groups. Compared with the control group, the AI of the second group (treated only with low-dose of the present invention drug) decreased dramatically (*P < 0.0001). Moreover, the AI of the fourth group (treated with bone marrow-derived mesenchymal stem cells transplantation combining with the drug of the invention for intervention) was obviously lower than the second group (treated only with low-dose the invention drug) (*P < 0.0001). But compared with the control group, the AI of the third group (treated only with bone marrow-derived mesenchymal stem cells transplantation for intervention) was of no significant difference (**P=0.289).

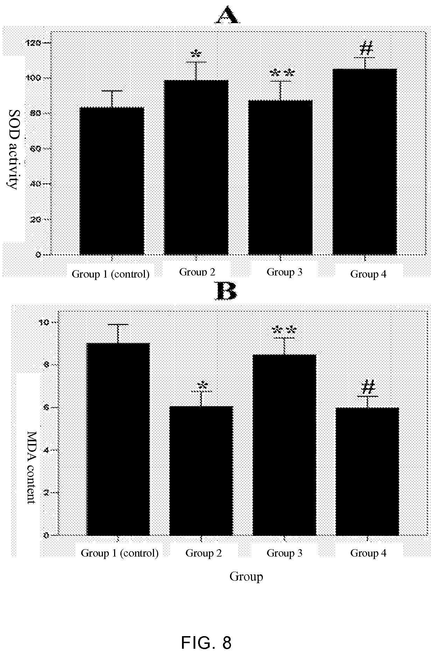

**Figure 8****. Detection of the myocardiac oxidative stress level surrounding the infarct zone at the end of the experiment.**

The superoxide dismutase (SOD) activities of the second group (treated only with low-dose of the drug of the invention for intervention) and the fourth group (treated with bone marrow-derived mesenchymal stem cells transplantation combining with the drug of the invention for intervention) increased significantly when compared with the control group (*P < 0.05, ^{#}P < 0.05), but there was no obvious difference between that of the third group (treated only with bone marrow-derived mesenchymal stem cells transplantation for intervention) and the control group (**P = 0.449). (B) The content of malondialdehyde (MDA) of the second group (treated only with low-dose of the drug of the present invention) and the fourth group (treated with bone marrow-derived mesenchymal stem cells transplantation combining with the drug of the invention for intervention) were significantly decreased compared with the control group (*P < 0.05, ^{#}P < 0.05). There was no significant difference between the third group (treated only with bone marrow-derived mesenchymal stem cells transplantation) and the control group (P = 0.195).

### Specific Embodiments

### EXAMPLE 1: Preparation of the drug of the invention

a) Formulation of raw materials:
   ginseng 55 g
   leech 103.75 g
   ground beetle 68.75 g
   olibanum (processed) 22.5 g
   red peony root 47.5 g
   rosewood heart wood 23.75 g
   sandalwood 22.5 g
   scorpion 68.75 g
   cicada slough 68.75 g
   centipede 13.75 g
   borneol 13.75 g
   spine date seed(stir-baked) 46.25 g;
b) pulverization process for medicinal materials:
   selecting and washing the five worm medicines of scorpion, leech, centipede, ground beetle and cicada slough, then according to prescription combining them with processed olibanum, crushing with crusher to obtain a coarse powder which can reach above 80 mesh; superfine grinding the coarse powder by using superfine pulverizing technologies to achieve medicinal powder in size less than 30-40 µm; prescribing the medicinal materials under pulverization after cleaning, drying and sterilizing;
c) Extraction, concentration and drying processes:
   adding water to rosewood heart wood and sandalwood, extracting volatile oils from them followed by extracting them with water, decocting red peony root and spine date seed twice, 3 hours for each time, combining the decoction, filtering it, then putting aside; extracting ginseng with suitable amount of 70% ethanol twice, 3 hours for each time, combining the ethanol solutions, recovering the ethanol completely, then extracting the residue of ginseng with water, concentrating the ethanol solution to ethanol extract of relative density of 0.9-1.1 (60°C), filtering the water solution of ginseng and combining it with all of the above water solutions, blending, concentrating the solution into water extract of relative density of 0.9∼1.1(60 °C), putting aside;
d) preparation process:
   feeding of the superfines into fluid bed granulating drier, then spraying the extract of step c) to granulate; finishing the granules, adding the fine powder of borneol, spraying volatile oils extracted from rosewood heart wood and sandalwood, filling with capsule filling machine to make into 1000 capsules after mixing well.

Dosage of the drug of the invention calculated from the total weight of the raw material as active component is 2-4 capsules each time, 3 times daily.

### Experimental Example: Promotional role of the drug of the invention in the application of the bone marrow-derived mesenchymal stem cells

### Materials and methods

### Animals

Chinese minipigs aged 10 months with body weight 30 kg ± 5kg were provided by Experimental Animal Center of China Agricultural University. All the animals were treated humanely, according to the U.S.A. National Institutes of Health issued "The Guide for management and use of laboratory animals". And all the experimental programs were supported by Animal Management Committee of Chinese Academy of Medical Sciences Laboratory, and approved by Experimental Animal Ethics Committee of Chinese Fu Wai Hospital, Peking Union Medical College.

### Isolation and culture of swinish bone marrow-derived mesenchymal stem cells

The swines were anesthetized by intramuscular injection of ketamine and diazepam at a dose of 25mg/kg and 1mg/kg, respectively. In an aseptic condition, the skins in the left iliac crest were prepared and draped, and cramp out 50ml of bone marrow with syringe containing 12,500 units of heparin. All the animals were given 0.3 mg buprenorphine by intramuscular injection before being returned to the breeding rooms.

The isolation and culture methods of bone marrow-derived mesen-chumal stem cells were made slight modifications according to the methods reported before. In short, the extracted bone marrow was diluted 1-fold with PBS, adding silica colloidal suspension (Percoll separation solution, 1.077g/ml, Sigma Company), 800 g centrifugal separating single nuclear cells for 30 minutes at 4 °C. After rinsed the cell precipitate with PBS twice, the cells were cultured at a density of 5 x 10⁵/cm² with normal medium (containing low glucose DMEM (Gibco Company), 10% fetal bovine serum (Gibco), 100U/ml penicillin and streptomycin) in wet incubator with 5% carbon dioxide at 37 °C. Three days later, removed hematopoietic cells, fibroblasts and other non-adherent cells by replacing the culture medium. The retained and purified adherent bone marrow-derived mesenchymal stem cells were cultured for further proliferation. The culture is replaced every 3 days during the experiment. After 10 days of culture, adherent cells formed a homogeneous cell clone. When reaching to 80% confluence, added 0.25% trypsin -0.02% EDTA solution (Sigma) to the adherent cells to resuspend it, at a passage efficiency of 1:3 for further culture.

### Preparation of myocardial infarction model and the transplanted cells and the promoter action of the drug of the invention

28 Chinese minipigs were divided into four groups: the first group was the control group (n=7), the second group (treated only with low-dose the drug of the invention, n=7), the third group (treated only with bone marrow-derived mesenchymal stem cells transplantation, n=7), the fourth group (treated with bone marrow-derived mesenchymal stem cells transplantation combined with the drug of the invention, n=7).

After the cells became 80% confluent, separated them from the culture flasks, re-suspended them in DMEM (GIBCO) containing 10% fetal bovine serum, labelled with 4',6-diamidino-2-phenylindole dihydrochloride (DAPI) (50µg/ml, Sigma) for 30 minutes at 37 °C. Rinsed the cells 6 times in PBS to wash off the non-bound DAPI, and then selected 3 ×10⁷ cells of each animal and place them into warm DMEM for several minutes before transplantation. The labelling process was very important which must ensure that all transplant nuclei are strained.

The swines were anesthetized by intramuscular injection of ketamine and diazepam at a dose of 25mg/kg and 1mg/kg, respectively, trachea cannula, connected mechanical respirator for artificial ventilation, maintained anesthesia by intravascular injection of ketamine and diazepam. Chest cutting along the midline of sternum, isolated coronary artery left anterior descending branch (LAD) to the first opposite angles branch, and ligated with a plastic annular tuber to ensure the formation of ischemic area. Intravenously injected 2mg/kg lidocaine before coronary artery ligation, intravenous administration should continue until the surgery end, the maintenance dose was 0.5mg/min. Blocked coronary artery left anterior descending branch (LAD) for 90 minutes to ensure the formation of myocardial infarction - reperfusion model.

The infarcted zones and their surrounding areas were injected 500µl suspension of autologous bone marrow-derived mesenchymal stem cells (3 × 10⁷ cells) 30 minutes after reperfusion. Animals in the control group were injected DMEM at the same volume.

After transplantation, closed thoracic incision, mediastinally placed an 18F catheter to rebuild the intrathoracic negative pressure and drained residual blood and irrigating solution. After these, discontinued the narcotics, ex-tubated the trachea cannula when appropriate to heal the wound. Removed the chest tube in the absence of gas leakage or residual blood. All animals received antibiotic therapy after surgery, intramuscularly injected cephalosporin V 1.0, twice daily for 3 days, while the animals were intramuscularly injected buprenorphine to relieve pain, twice daily, 0.3mg each time, for 3 days.

The animals received the drug of the invention for intervention treatment at a dosage based on the previous experiment from 3 days before to 4 days after bone marrow-derived mesenchymal stem cells tranplantation, the dose was 0.05 g•kg•d.

### Magnetic resonance imaging (MRI)

One and six weeks after tranplantation, the parameters of cardiac function of the experimental animals were acquired by Cine MRI and enhanced MRI, respectively. Magnetic resonance imaging was performed by clinically used 1.5T MRI scanner [Siemens, Germany] with RF coil. The experimental animals were anesthetized by intramuscular injection of ketamine and diazepam, the dose was 25mg/kg and 1mg/kg, respectively. MRI used wireless ECG-gated spin echo. Cine MRI and corresponding enhanced MRI scanned one layer every 4mm, and there were 6-8 layers beginning at the valvula bi-cuspidalis level. Detected the horizontal and sagittal view to determine the right planes of the short axis, determined an imagine of the long axis every 60°.The film MRI imagines were acquired by steady-state fast gradient echo (TrueFisp) combined with pulse sequence sensitive encoding (TSENSE) parallel technology. The parameters of typical imagines were as followings: Cycle time (TR) = 41.7 ms, echo time (TE) = 1.39 ms, the bandwidth (BW) = 965 Hz / PX, flip angle (FA) = 48 °, image matrix = 109 × 192, spatial resolution = 3.2mm × 2.0 mm, slice thickness (SL) = 6.0 mm, parallel factor = 3. Echo imaging device by TSENSE imaging technology was used to obtain the first flow perfusion images of the 3-4 short axis and a four chamber perfusion images (TR = 6.0 ms, TE = 1.22 ms, FA = 30 °, spatial resolution = 2.8 mm × 2.8 mm, SL = 10.0 mm, parallel factor = 2, 4-5 images per heartbeat, all the levels were pulses before the first saturation).The first scan obtained image about 60 cardiac cycles. Rinsed 0.1 mmol Gd-DTPA (Schering AG) with 20 mL 0.9% NaCl (flow rate 4mL/s). Injected 0.1mmol/kg Magnevist solution intravenously after perfusion intravenous, 5 minutes later injected 0.2mmol/kg diethylenetriamine pentaacetic acid gadolinium (Gd-DTPA) intravenously, and then directly took enhanced MRI photography. Inversion recovery (PSIR) Flash sequence was used to carry out T1 weighting, PSIR was used to adjust T1. Typical image parameters were as follows: TR = 700ms, TE = 4.8ms, BW = 130 kHz, plane resolution = 1.8 × 1.3mm, image matrix = 156 × 256, SL = 8mm. Re-shooted all the films MRI and enhanced MRI images 6 weeks after stem cell transplantation. Ensure the short axis sections in accordance with the baseline firstly shot according to the anatomic regionalization. In addition, to provide a healthy control, five sham animals were investigated using the same experimental design of MRI.

### Single photon emission computed tomography (SPECT)

Myocardial single photon emission computed tomography was taken at one and six weeks after cell transplantation to detect the myocardial perfusion defect area. Intravenous injection of ^{99m} 296MBq (8mCi) Tc-methoxy isobutyl isonitrile, took the myocardial SPECT with y camera after 45-60 minute. Using low-energy dual-head y camera (Varicum, GE) with high-resolution collimator with 20% energy windows, set at the 140KeVy peak. Acquired 40s, 32 images each frame, acquired matrix 64 × 64, projection range from right anterior oblique 45 ° ∼ left posterior oblique 45 °, a total of 180 °. SPECT was reconstructed with Butterworth low filter, cutoff frequency was 0.45, the type was 5, by adjusting the cardiac shaft to rebuild the image data of the short axis, vertical long axis, and the horizontal long axis, the three axes plane. Perfusion defect area was calculated by flash method bulls eye technics.

### Histological analysis

To detect the potentiality of transplanted bone marrow-derived mesenchymal stem cells differentiating into cardiomyocytes and vascular, frozen tissue sections of cardiac was analyzed by fluorescent immunoassay at 5µm thickness by serially sectioning. The antibodies for detection included: vascular endothelial cell-specific factor VIII (VWF 1:50, DAKO), α-smooth muscle actin (SM-actin, 1:50, DAKO), α-skeletal muscle actin (1: 50, DAKO), cardiac troponin T (cTn-T, 1:50, Sigma), connexin 43 (1:50, Sigma). After rinsing the sections with PBS, incubated by rhodamine or fluorescein isothiocyanate-labelled goat anti mouse (GAM) or rabbit IgG. Finally, took pictures by laser scanning confocal microscope.

Determination the survival and differentiation potential of bone marrow-derived mesenchymal stem cell transplanted *in vivo,* left ventricle was cut into 8 pieces from the apex to the bottom at the cross-sectional, randomly selected five 5-µm thick frozen sections of each piece. Under the fluorescence microscope, randomly selected five horizons of each frozen section to count the DAPI and cTn-T positive cells. The cTn-T positive cells were considered to be going to differentiate into cardiomyocytes. Randomly selected five sections in the infarction to detect the intercellular staining intensity of connexin 43, analyzed with image analysis system.

Detected the capillary density in the infarcted zone and the surrounding zones, the method of the preparation of tissue was described in Weidner N, Semple JP, Welch WR, Folkman J. Tumor angiogenesis and metastasis-correlation in invasive breast carcinoma (N Engl J Med 1991; 324:1 - 8) [PMID: 1701519]. The sections were stained with VWF antibody (1:200, DAKO). Selected five and eight sections from infarction and surrounding zones of each experimental animal, respectively, the sections were analyzed by a research staff who did not involve in the treatment of the cells to count positive staining blood vessels. Selected 5 high power field of each section to count, the results were shown as the number of capillaries under each high power field.

### Deoxynucleotidyl transferase-mediated deoxy uridine triphosphate (dUTP) nick end labeling (TUNEL) to detect apoptosis

We used TUNEL analysis (Roche, Germany) to detect cellular apoptosis in the myocardial tissue. At the end of the experiment, we obtained the sections around the area of infarcted tissue of all the animals, the paraffin sections were dewaxed with trypsinization, incubated with dUTP labeled terminal deoxynucleotidyl transferase (TdT) and fluorescein at 37 °C in wet box for 60 minutes. Then incubated with alkaline phosphatase specific antibody conjugated hydrofluorescin for 30 minutes, 3,3-diaminobenzidine (DAB) was used for color staining to TUNEL staining, the nuclei containing DNA broken segments were stained blue. In order to detect the ratio of apoptotic nuclei in the sections, the tissue sections were counterstained with cardiac specific Desmin monoclonal antibody (1:100, DAKO), the tissue sections were observed under microscope at 400 times, counted more than100 cardiac cells at least in eight high power field, apoptosis index refers to the percentage of the number of the apoptotic myocardial cells to the total number of cardiocytes in the field of vision.

### Antioxidant enzyme activity and lipid peroxides

In order to detect the level of oxidative stress in myocardial infarction, we obtained the myocardial tissue surrounding the infraction in the experimental end, detected the superoxide dismutase by the method of xanthine oxidase (Nanjing Jiancheng Company), lipid peroxides was expressed as the level of myocardial MDA detected by the method of thiobarbituric acid (Nanjing Jiancheng Company).

### Statistical analysis

The continuous variable were expressed as mean ± standard deviation, chi-square test (χ2) was used to analysis the rate of difference between the third and the fourth group. After homogeneity of variance and normality test were performed, then carried out the analysis of variance to determine the difference of each group in each phase (the baseline was tested one week after transplantation, six weeks after transplantation was the end point detection), analyzed the data of six weeks after transplantation referring to the data of one week after transplantation. Various parameters were compared between the two groups by least significant difference (LSD). The data were corrected by Bonferroni method, when P <0.05, the difference had the remarkable significant. All data were analyzed by SPSS13.0.

### Results

Before successfully collected all the parameters, there was one animal dead in the control group, the second group, and the third group, respectively, the data of the dead animals were not included in the statistical analysis.

### Histological analysis

6 weeks after cell transplantation, HE staining showed that in the control group, the infarction emerged severe fibrosis with chronic inflammatory cell infiltration, myocardial cell survival rarely, the situation of the second and the third group was the same. In contrast, to the fourth group it found mild fibrosis and chronic inflammatory cell infiltration, and there was some survival myocardial cells in infarction (Figure 1).

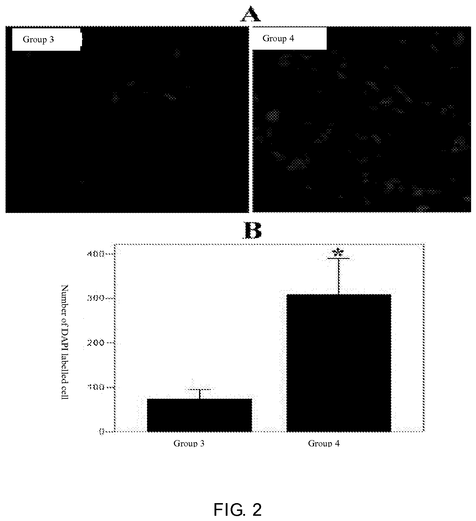

It was observed that the positive cells labelled DAPI of the fourth group were obviously more than that of the third group (308.9±88.2 versus 73.2±21.3, P <0.0001) (Figure 2A-B).

Six weeks after transplantation, immunofluorescence analysis of the third and the fourth groups showed that the positive cells labelled DAPI expressed myocardial-specific and microvascular-specific proteins, including α-skeletal muscle actin, cardiac troponin T, von Willebrand factor, and vascular smooth muscle actin, indicating that portions of the implanted bone marrow-derived mesenchymal stem cells have differentiated into cardiac muscle and micrangium (Figure 3A-C). Especially to the fourth group, the differentiation rate of implanted bone marrow-derived mesenchymal stem cells into myocardial cells was significantly higher than that of the third group (45.8±5.1% versus 8.7±2.4%, P <0.0001) (Fig. 3 D).

In addition, the intercellular connection of the positive cells labelled DAPI in infractions was studied by detecting the expression of connexin 43. The results showed that the expression of connexin 43 of the fourth group was significantly more than that of the third group (16.1±1.4 versus 4.7±1.8, P <0.0001) (Figure 4A, B).

### Capillary density

The capillary density of the infarction and the border zones were determined according to VWF antibody immunohistochemical staining, there was no significant difference of the capillary density of the control group when compared with the second group and the third group (1.8±0.5/HPF versus 2.0± 0.6 versus 1.8±0.8, P> 0.05). However, compared with the third group, the capillary density of the infraction of the fourth group increased by 105% (3.7± 1.0/HPF, P <0.0001). The capillary density of infarction border zone of the fourth group was 8.9 ± 1.9/HPF, which was significantly higher than the other three groups (4.9±1.3/HPF, 5.1 ± 0.9, 5.2±1.4, P <0.0001) (Figure 5).

### Magnetic resonance imaging and single photon emission computed tomography

36 segments were chosen from each group to analyze, counted the movement disorders segment to calculate the rate of wall thickening. One week after transplantation the movement disorders segment of the 36 segments of the control group, the second group, the third group, and the fourth groups was 8.2±3.0, 8.3±3.1, 8.7±3.9, and 8.9±3.6, respectively, accounting for 22.8%, 23.1%, 24.2% and 24.7% of the total, respectively, there was no significant difference in every group (P = 0.983). All segments of movement disorders were used to measure the rate of the regional wall thickening. One week after transplantation, the other parameters, including rate of regional wall thickening (P = 0.915), left ventricular ejection fraction (LVEF, P = 0.996), infarct size (P = 0.991), left ventricular end-diastolic volume (EDV, P = 0.852), left ventricular end systolic volume (ESV, P = 0.990), left ventricular mass index (LVmass index, P = 0.791), among the 4 groups there was no significant difference. Six weeks after transplantation, the parameters of cardiac function of the fourth group, except EDV and ESV, were significantly improved (P <0.0001) as compared with the control group. The left ventricular function and geometry changes in the ventricular see Table 1.

**Table 1. The MRI results of left ventricular function and structure one and six weeks after transplantation**

| Group | 1 | | 2 | | 3 | | 4 | |
|---|---|---|---|---|---|---|---|---|
| | baseline | endpoint | baseline | endpoint | baseline | endpoint | baseline | endpoint |
| LVEF (%) | 42.6±7.9 | 43.9±7.6 | 43.3±7.9 | 44.8± 8.5 | 43.5±10.0 | 45.7±9.6# | 42.7±7.6* | 50.0±10.1## |
| EDV (ml) | 57.8±5.8 | 66.2±6.8 | 58.2±5.8 | 65.8±5.6 | 56.8±6.9 | 63.5±6.3# | 55.1±8.2* | 64.6±7.5 ** |
| ESV (ml) | 33.5±7.6 | 37.3±7.5 | 33.3± 7.7 | 36.7±8.3 | 32.7±9.6 | 35.0±9.5# | 32.1±9.2* | 32.8±10.1** |
| Dyskinetic Segments | 8.2±3.0 | 7.7±2.4 | 8.3±3.0 | 7.5±2.3 | 8.7±3.9 | 7.8±3.4# | 8.9±3.6* | 4.9±1.8## |
| Wall thickness (%) | -25.5±17.2 | -27.5±15.7 | -27.2±15.6 | -25.7±14.0 | -23.0±14.5 | -20.2±12.8# | -25.9±14.1* | 35.9±10.8## |
| Infarct size (cm²) | 6.6±2.0 | 6.7±2.1 | 7.0±2.2 | 7.1±2.3 | 6.8±2.1 | 7.0±2.1# | 6.5±2.3* | 3.3±1.8## |
| LV mass Index (g/m²) | 64.7±6.3 | 77.8±8.1 | 63.2±8.1 | 76.2±8.1 | 60.2±7.9 | 76.0±5.4 # | 60.8±.4* | 66.4±8.1## |

The baseline represents one week after transplantation; the endpoint refers to six weeks after transplantation; LVEF is fraction of left ventricular ejection; EDV is the volume of left ventricular end diastolic volume; ESV is the volume of left ventricular end systolic volume; Dyskinetic segments; Wall thickness (%); Infarct size; left ventricular mass index (LV mass index); * P> 0.05 (compared among the four groups one week after transplantation); # P> 0.05 (the control group compared with the second group six weeks after transplantation); ** P> 0.05 (the end-diastolic volume and end systolic volume of the second group and the third group six weeks after transplantation compared with that of the control group); ## P <0.0001 (the second group and the third group compared with the control group six weeks after transplantation)

Figure 6 comprises the typical SPECT images for detection of perfusion defect area one and six week after transplantation. The SPECT results of the initial first week after transplantation showed there was no significant difference among the four groups (50.7±14.5% versus 52.7±15.5% versus 51.8±16.5% versus 49.4±16.0%, respectively, P = 0.984). Six weeks after transplantation, SPECT results of the experimental endpoint showed the average perfusion defect size of the control group, the second group, and the third group became 47.8±11.1%, 50.7±12.5%, 47.3±13.2% (n = 6, P = 0.899), while the average area of perfusion defects of the fourth group was 22.1± 9.3%, which reduced significantly compared with the first three groups (n = 7, P <0.0001).

### Cellular apoptosis around the infarcted myocardium

By staining myocardial cell specific marker binding proteins accompanied with DNA end labelling, apoptotic cells of the left ventricular infarction of the composition of the invention, including the second group and the fourth group were significantly reduced compared with that of the control group (apoptotic index 6.1±1.4, 2.4±0.9 compared to 10.1±1.8, P <0.0001), and the apoptotic index of the fourth group was also significantly less than that of the second group (P <0.0001). However, the apoptosis index of the third group showed no significant difference compared with the control group (P = 0.289). (Figure 7)

### Assessment of the level of oxidative stress

At the end of the experiment, the SOD activities in peripheral myocardial infarction of the second group and the fourth group were significantly higher than that of the control group (98.7±9.8, 105.1±7.0 to 83.4±8.8 U/mg protein, P <0.05), it showed that the drug treated group could enhance the free radical scavenging activity, however, there was no significant difference between the third group and the control group (87.4±10.2 U/mg protein, P = 0.449). With corresponding the MDA levels in myocardial infarction of the second group and the fourth group were markedly decreased (6.1±0.7, 6.0±0.6 versus 9.0±0.8 nmol/mg protein, P <0.05), indicating that the drug of the inventions could significantly alleviate lipid peroxidation and cell damage caused by it, there was no significant difference between the control group and the third group (8.5±0.8 nmol/mg protein, P = 0.195) (Figure 8).

### Discussion

The results of the experiment showed that: (1) Injecting autologous bone marrow-derived mesenchymal stem cells immediately after acute myocardial infarction/reperfusion, the survival and differentiation abilities of transplanted cells were limited, which did not contribute significant benefit to the cardiac function; (2) Use of low dose of the present invention for a short-term will not produce significant benefits to cardiac function too, however based on the use of the present drug, inject bone marrow-derived mesenchymal stem cells immediately after acute myocardial infarction/reperfusion, the survival and differentiation abilities of transplanted cells significantly enhanced when compared with the group only transplanted cells *in vivo.* In addition, the drug of the invention combined with stem cells transplantation can also reduce infarct size, promote angiogenesis, improve cardiac function, and reverse ventricular remodelling.

The most important finding of this experiment is that based on the treatment of low-dose of the composition of the invention short term, injecting bone marrow-derived mesenchymal stem cells into myocardium immediately after acute myocardial infarction and reperfusion, the survival and differentiation ability of implanted cells *in vivo* has enhanced significantly than that of the group only implanted bone marrow-derived mesenchymal stem cells, and also improved the heart function significantly at the same time. It suggests that transplantation, survival and differentiation of stem cell show a strong dependence on the myocardial micro-environment after acute infarction.

This experiment showed that cell survival and differentiation of the combination of the drug of the invention and bone marrow-derived mesenchymal stem cell transplantation has significantly improved when compared with simply transplantation of bone marrow-derived mesenchymal stem cells group. In addition, use of low doses of the present composition alone did not significantly improve cardiac function. Based on the above results, we could infer that the improvement of the regional micro-environment by the drug of the invention group after acute myocardial infarction enhance the survival rate and biological activity of the implanted cells. Despite alone using of low-dose of the drug of the invention would not produce significant effects, but it can significantly improve the survival and differentiation of the implanted bone marrow-derived mesenchymal stem cells. The experimental results showed that short-term use of low-dose of the drug of the invention could promote the cardiomyoplasty by implantation of autologous bone marrow mesenchymal cells. Detection of the gene expression profiles after cardiac infarction by microarray gene chip technology found that using low-dose of the drug of the invention alone could result in positive changes to gene expression, including up-regulation of anti-inflammatory, anti-apoptosis, anti-fibrosis gene (data not shown). Based on the above studies, we believe that use of low dose of the drug of the inventions after acute myocardial infarction for a short-period could improve the regional myocardial micro-environment after acute infarction, so that make the implanted bone marrow-derived mesenchymal stem cells stably survive and differentiate.

In summary, we firstly found that using low dose of the drug of the inventions short period could effectively improve the regional micro-environment after acute myocardial infarction, promote cell cardiomyoplasty by implantation of autologous bone marrow-derived mesenchymal stem cells, and it is of significance to clinical application of bone marrow stromal stem cell transplantation.

## Claims

1. A traditional Chinese medicinal composition for use in the treatment or prevention of cardiovascular disease, **characterized in that** the traditional Chinese medicinal composition is composed of the following crude drugs (by wt. portions):
ginseng 6
leech 11
ground beetle 7
olibanum (processed) 2
red peony root 5
rosewood heart wood 2
sandalwood 2
scorpion 3
cicada slough 7
centipede 1
borneol 5
spine date seed (stir-baked)5.

2. A traditional Chinese medicinal composition for use in the treatment or prevention of cardiovascular disease, **characterized in that** the traditional Chinese medicinal composition is composed of the following crude drugs (by wt. portions):
ginseng 5.5
leech 10.375
ground beetle 6.875
olibanum (processed) 2.25
red peony root 4.75
rosewood heart wood 2.375
sandalwood 2.25
scorpion 6.875
cicada slough 6.875
centipede 1.375
borneol 1.375
spine date seed (stir-baked) 4.625.

3. The composition for use according to claim 1 or 2, wherein the active ingredients of the traditional Chinese medicinal composition are composed of the following ingredients:
a. scorpion, leech, centipede, ground beetle, cicada slough and processed olibanum powder, which has a mean particle size of less than 100 µm;
b. borneol powder;
c. volatile oils extracted from rosewood heart wood and sandalwood;
d. condensed alcohol extracts from ginseng extracted with ethanol;
e. condensed water extract, which is obtained as following: extracting the residue of rosewood heart wood and sandalwood with water after extracting component c from them, decocting red peony root and stir-baked spine date seed with water, extracting the residue of ginseng with water after extracting component d from it, filtering all of the above water extracts, blending them, then concentrating.

4. The composition for use according to any one of claims 1 to 3 in form of capsule, tablet, pill, oral liquid, soft capsule, or guttate pill.

5. The composition for use according to any one of claims 1 to 4, **characterized by** the use of the traditional Chinese medicinal composition as a medicament to treat cardiovascular disease in combination with autologous bone marrow-derived mesenchymal stem cell.

6. The composition for use according to any one of claims 1 to 5, wherein the cardiovascular disease is myocardial infarction.

7. The composition for use according to any one of claims 1 to 5, wherein the cardiovascular disease is acute myocardial infarction.

## Patentansprüche

1. Traditionelle chinesische medizinische Zusammensetzung für den Gebrauch bei der Behandlung oder Verhütung kardiovaskulärer Erkrankungen, **dadurch gekennzeichnet, dass** die traditionelle chinesische medizinische Zusammensetzung aus den folgenden Roharzneimitteln (in Gewichtsanteilen) besteht:
Gingseng 6
Blutegel 11
Laufkäfer 7
Olibanum (verarbeitet) 2
rote Pfingstrosenwurzel 5
Palisander-Herzholz 2
Sandelholz 2
Skorpion 3
abgestreifte Zikadenhaut 7
Tausendfüßler 1
Borneol 5
Spine Datum-Samen (unter Rühren gebacken) 5.

2. Traditionelle chinesische medizinische Zusammensetzung für den Gebrauch bei der Behandlung oder Verhütung kardiovaskulärer Erkrankungen, **dadurch gekennzeichnet, dass** die traditionelle chinesische medizinische Zusammensetzung aus den folgenden Roharzneimitteln (in Gewichtsanteilen) besteht:
Gingseng 5,5
Blutegel 10,375
Laufkäfer 6,875
Olibanum (verarbeitet) 2,25
rote Pfingstrosenwurzel 4,75
Palisander-Herzholz 2,375
Sandelholz 2,25
Skorpion 6,875
abgestreifte Zikadenhaut 6,875
Tausendfüßler 1,375
Borneol 1,375
Spine Datum-Samen (unter Rühren gebacken) 4,625.

3. Zusammensetzung für den Gebrauch nach Anspruch 1 oder 2, wobei die aktiven Zutaten der traditionellen chinesischen medizinischen Zusammensetzung aus den folgenden Zutaten bestehen:
a. Skorpion, Blutegel, Tausendfüßler, Laufkäfer, abgestreifte Zikadenhaut und verarbeitetes Olibanumpulver, das eine mittlere Teilchengröße von kleiner als 100 µm hat;
b. Borneolpulver;
c. flüchtige Öle, die aus Palisander-Herzholz und Sandelholz extrahiert sind;
d. kondensierte Alkoholextrakte aus Gingseng, die mit Ethanol extrahiert sind;
e. kondensierter Wasserextrakt, der wie folgt erhalten wird: Extrahieren des Rests von Palisander-Herzholz und Sandelholz mit Wasser nach dem Extrahieren der Komponente c aus diesen, Abkochen der roten Pfingstrosenwurzel und des unter Rühren gebackenen Spine Datum-Samens mit Wasser, Extrahieren des Rests von Gingseng mit Wasser nach dem Extrahieren der Komponente d daraus, Filtern der gesamten oben genannten Wasserextrakte, Mischen dieser, danach Konzentrieren.

4. Zusammensetzung für den Gebrauch nach einem der Ansprüche 1 bis 3 in der Form einer Kapsel, Tablette, Pille, oralen Flüssigkeit, Weichkapsel oder Guttate-Pille.

5. Zusammensetzung für den Gebrauch nach einem der Ansprüche 1 bis 4, **gekennzeichnet durch** den Gebrauch der traditionellen chinesischen medizinischen Zusammensetzung als ein Medikament zum Behandeln kardiovaskulärer Erkrankung kombiniert mit autologer von Knochenmark abgeleiteter mesenchymaler Stammzelle.

6. Zusammensetzung für den Gebrauch nach einem der Ansprüche 1 bis 5, wobei die kardiovaskuläre Erkrankung Myokardinfarkt ist.

7. Zusammensetzung für den Gebrauch nach einem der Ansprüche 1 bis 5, wobei die kardiovaskuläre Erkrankung akuter Myokardinfarkt ist.

## Revendications

1. Composition de médecine chinoise traditionnelle pour utilisation dans le traitement ou la prévention d'une maladie cardiovasculaire, **caractérisée en ce que** la composition de médecine chinoise traditionnelle est composée des médicaments bruts suivants (en parties en poids) :
ginseng 6
sangsues 11
carabe 7
oliban (transformé) 2
racine de pivoine rouge 5
coeur de bois de rose 2
bois de santal 2
scorpions 3
exuvie de cigale 7
mille-pattes 1
bornéol 5
graine de datte épineuse (frite) 5.

2. Composition de médecine chinoise traditionnelle pour utilisation dans le traitement ou la prévention d'une maladie cardiovasculaire, **caractérisée en ce que** la composition de médecine chinoise traditionnelle est composée des médicaments bruts suivants (en parties en poids) :
ginseng 5,5
sangsues 10,375
carabe 6,875
oliban (transformé) 2,25
racine de pivoine rouge 4,75
coeur de bois de rose 2,375
bois de santal 2,25
scorpions 6,875
exuvie de cigale 6,875
mille-pattes 1,375
bornéol 1,375
graine de datte épineuse (frite) 4,625.

3. Composition pour utilisation selon la revendication 1 ou 2, dans laquelle les substances actives de la composition de médecine chinoise traditionnelle sont composées des substances suivantes :
a. scorpions, sangsues, mille-pattes, carabe, exuvie de cigale et poudre d'oliban transformé, qui a une taille moyenne de particule inférieure à 100 µm ;
b. poudre de bornéol ;
c. huiles volatiles extraites de coeur de bois de rose et de bois de santal ;
d. extraits condensés dans de l'alcool de ginseng extrait avec de l'éthanol ;
e. extrait condensé dans de l'eau, qui est obtenu comme suit :
extraction du résidu de coeur de bois de rose et de bois de santal avec de l'eau après extraction du composant c de ceux-ci, décoction de racine de pivoine rouge et de graine de datte épineuse frite avec de l'eau, extraction du résidu de ginseng avec de l'eau après extraction du composant d de celui-ci, filtration de tous les extraits dans de l'eau ci-dessus, mélange de ceux-ci, puis concentration.

4. Composition pour utilisation selon l'une quelconque des revendications 1 à 3 sous la forme d'une capsule, d'un comprimé, d'une pilule, d'un liquide oral, d'une capsule molle ou de gouttes.

5. Composition pour utilisation selon l'une quelconque des revendications 1 à 4, **caractérisée par** l'utilisation de la composition de médecine chinoise traditionnelle en tant que médicament pour traiter une maladie cardiovasculaire en association avec une cellule souche mésenchymateuse issue de moelle osseuse autologue.

6. Composition pour utilisation selon l'une quelconque des revendications 1 à 5, dans laquelle la maladie cardiovasculaire est un infarctus du myocarde.

7. Composition pour utilisation selon l'une quelconque des revendications 1 à 5, dans laquelle la maladie cardiovasculaire est un infarctus aigu du myocarde.
